# EUROPEAN PATENT SPECIFICATION

(11) **EP 1 806 763 B1**
(45) Date of publication and mention of the grant of the patent: **25.04.2012**
(21) Application number: 07000196.1
(22) Date of filing: 05.01.2007
(51) Int. Cl.: H01J 17/49, H01J 17/16

(54) **Plasma display panel**
Plasmaanzeigetafel
Panneau d'affichage à plasma

(30) Priority: 05.01.2006 KR 20060001250
(43) Date of publication of application: 11.07.2007
(73) Proprietor: LG ELECTRONICS INC., Yeondeungpo-gu Seoul 150-875 (KR)
(72) Inventor: Ahn, Sungyong, Chilgok-gun Gyeongsangbuk-do (KR)
(74) Representative: von Hellfeld, Axel

(56) References cited:
- EP-A- 0 159 410
- EP-A- 1 187 166
- EP-A1- 1 596 410
- WO-A-2005/042427
- US-A1- 2004 235 385
- US-B1- 6 566 812

## Description

The present invention relates to a plasma display panel.

In general, a plasma display panel includes a phosphor layer within discharge cells partitioned by barrier ribs, and a plurality of electrodes supplied a driving signal to generate a discharge within the discharge cells.

In such a plasma display panel, when a driver supplies the driving signal to the plurality of electrodes, vacuum ultraviolet rays are produced due to a discharge gas filled into the discharge cells. The vacuum ultraviolet rays cause phosphors within the discharge cells to emit light, thereby implementing an image.

EP 1 187 166 A2 discloses a plasma display panel according to the preamble of claim 1, comprising a front substrate, a rear substrate formed in parallel to the front substrate, a first barrier rib and a second barrier rib, the first barrier rib formed between the front substrate and the rear substrate in a first direction, the second barrier rib formed in a second direction intersecting with the first direction, a black layer formed in the front substrate corresponding to the first barrier rib, wherein the first barrier rib and the second barrier rib partition a unit discharge cell such that the first barrier rib partitions unit discharge cells emitting the same color of light, such that the second barrier rib partitions unit discharge cells emitting different colors of light and such that a unit discharge space inside of the unit discharge cell is defined, wherein the area of the unit discharge space is equal to a first distance between top portions of the two neighboring first barrier ribs multiplied by a second distance between top portions of the two neighboring second barrier ribs, wherein the area of the unit discharge cell is equal to a third distance between central points of widths of top portions of the two neighboring first barrier ribs multiplied by a fourth distance between central points of widths of top portions of the two neighboring second barrier ribs, and wherein a ratio of the area of the unit discharge space to the area of the unit discharge cell ranges from about 0.45 to about 0.59.
Further, US patent 6,566,812 B1 discloses discloses a plasma display panel similar to the panel shown in EP 1 187 166 A2.

There is provided a plasma display panel according to claim 1. Further developments are as set forth in the dependent claims.

It is to be understood that both the foregoing general description and the following detailed description are exemplary and explanatory and are intended to provide further explanation of the invention as claimed.

The invention will be described in detail with reference to the following drawings in which like numerals refer to like elements.

FIG. 1 illustrates a plasma display panel structure.

FIGS. 2a to 2c illustrate sectional views of a plasma display panel comprising black layers positioned in non-discharge spaces.

FIG. 3 is a perspective view illustrating a structure of discharge cells of a plasma display panel according to an embodiment of the present invention.

FIG. 4a illustrates a graph of emission efficiency with respect to a ratio of the area of a unit discharge space to the area of a unit discharge cell.

FIG. 4b illustrates a graph of external light reflectivity of a plasma display panel with respect to a ratio of the area of a unit discharge space to the area of a unit discharge cell.

FIG. 5 illustrates a top view of a plasma display panel structure according to an embodiment of the present invention.

FIGS. 6a and 6b are perspective views illustrating a structure of a barrier rib of a plasma display panel according to an embodiment of the present invention.

The invention as defined in claim 1 will be described in detail with reference to the following drawings in which like numerals refer to like elements.

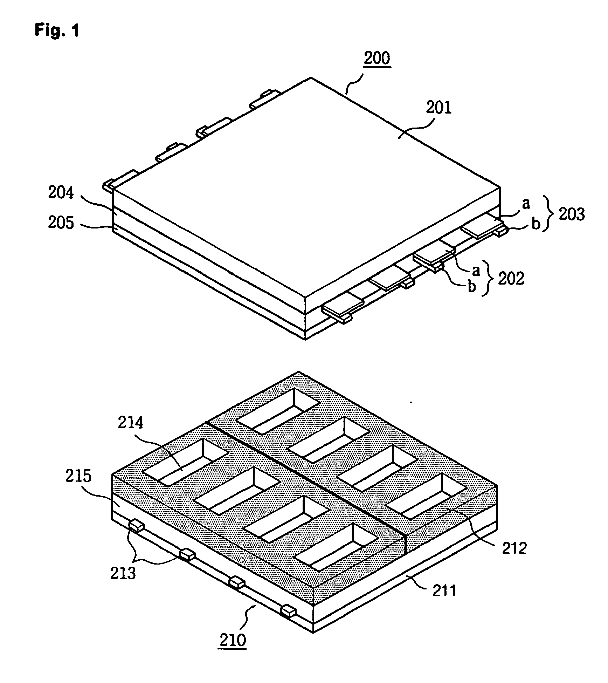

FIG. 1 illustrates a plasma display panel structure.

In the plasma display panel illustrated in FIG. 1, a front panel 200 and a rear panel 210 are adjoined together to run parallel with a given distance therebetween. The front panel 200 comprises a front substrate 21 on which an image is displayed, and scan electrodes 202 and sustain electrodes 203, which are formed on the front substrate 201. The rear panel 210 comprises a rear substrate 211, and address electrodes 213 arranged to intersect with the scan electrodes 202 and the sustain electrodes 203.

Each of the scan electrodes 202 and the sustain electrodes 203 formed on the front substrate 201 comprises a transparent electrode 'a' and a bus electrode 'b'. The transparent electrodes 'a' and the bus electrodes 'b' are formed of an indium-tin-oxide (ITO)-based material and a non-transparent metal-based material, respectively.

An upper dielectric layer 204 is formed on the front substrate 201 where the scan electrodes 202 and the sustain electrodes 203 are formed. The upper dielectric layer 204 limits discharge current of the scan electrodes 202 and the sustain electrodes 203, and insulates the scan electrodes 202 and the sustain electrodes 203 from each other.

A protection layer 205 is formed on the upper dielectric layer 204 to provide a readily discharging condition. The protection layer 205 is formed by depositing magnesium oxide (MgO) on the upper dielectric layer 204.

A lower dielectric layer 215 is formed on the rear substrate 211 where the address electrodes 213 are formed. The lower dielectric layer 215 insulates the address electrodes 213 from each other.

Barrier ribs 212 are formed on the lower dielectric layer 215 to partition discharge cells.

In such a case, the discharge cells partitioned by the barrier ribs 212 may be formed in a stripe-type, well-type, a delta-type, or a honeycomb-type structure.

Phosphor materials 214 are formed within the discharge cells partitioned by the barrier ribs 212 to represent colors including red (R), green (G) and blue (B). In addition to the R, G, and B discharge cells, phosphor materials for representing white W or yellow Y may be formed within the discharge cells.

Although FIG. 1 illustrates one exemplary plasma display panel, it should be noted that the plasma display panel structure is not limited to the illustrated exemplary structure. For instance, although the upper dielectric layer 204 and the lower dielectric layer 205 in the present embodiment are illustrated in a single layer structure, the upper dielectric layer 204 and the lower dielectric layer 205 may be formed in a multiple layer structure.

Also, it is illustrated in the present example that the front panel 200 comprises only the scan electrodes 202 and the sustain electrodes 203, and the rear panel 210 comprises only the address electrodes 213. However, the scan electrodes 202, the sustain electrodes 203, and the address electrodes 213 may be formed on the front panel 200. Alternatively, one of the scan electrodes 202, the sustain electrodes 203, and the address electrodes 203 may be formed on the barrier ribs 212.

The scan electrodes 202 and the sustain electrodes 203 may comprise merely the bus electrodes 'b' without the transparent electrodes 'a'.

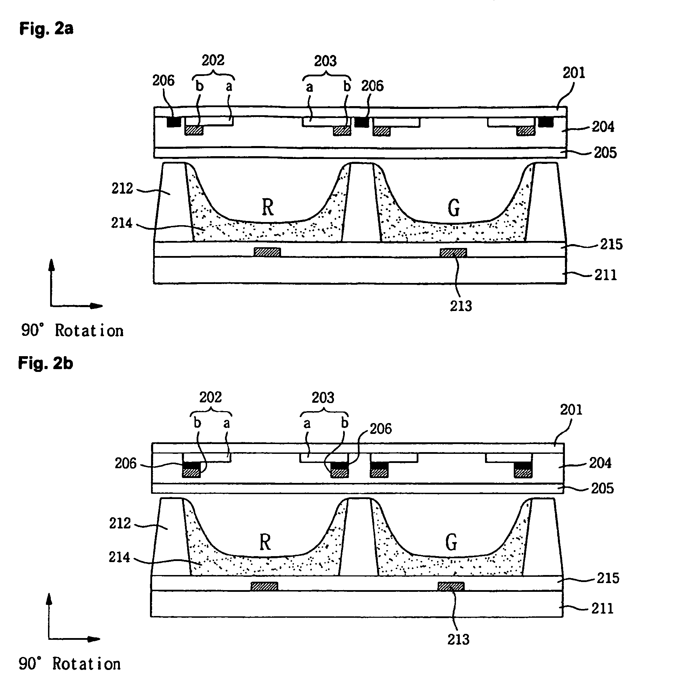

FIGS. 2a to 2c illustrate sectional views of a plasma display panel comprising black layers.

Referring to FIG. 2a, the black layers 206 are formed on regions of a front substrate 201 corresponding to barrier ribs 212 formed over a rear substrate 211. The black layers 206 are formed on the same surface of the front substrate 201 where scan electrodes 202 and sustain electrodes 203 are formed.

The black layers 206 may be formed inside an upper dielectric layer 204. In this case, although not illustrated, the upper dielectric layer 204 comprises a first upper dielectric layer and a second upper dielectric layer. The black layers 206 are formed to cover the scan electrodes 203 and the sustain electrodes 203, and formed in regions corresponding to barrier ribs 212 formed over the first upper dielectric layer. The second upper dielectric layer is formed on the first upper dielectric layer to cover the black layers 206.

Referring to FIG. 2b, the black layers 206 each are formed between a transparent electrode 'a' and a bus electrode 'b' for use in the scan electrodes 202 and the sustain electrodes 203. In this case, the black layers 206 are formed of a conductive material in order to make the transparent electrodes 'a' and the bus electrodes 'b' electrically coupled to each other. Also, the black layers 206 may be formed to integrate with the bus electrodes 'b' formed on the front substrate 201.

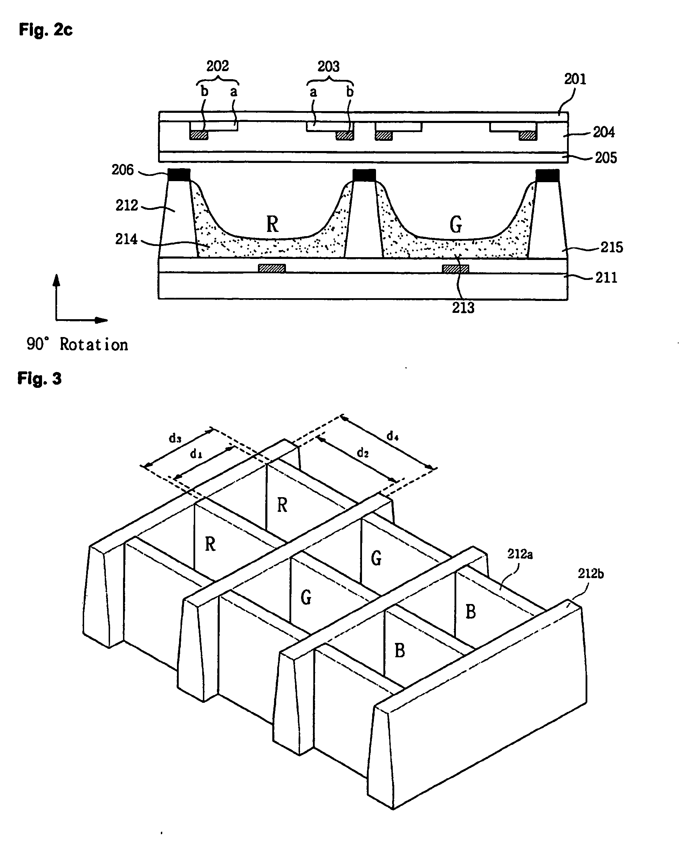

Referring to FIG. 2c, the black layers 206 are formed on the barrier ribs 212. The black layers 206 are formed to integrate with the barrier ribs 212.

The black layers 206 of the plasma display panel are formed in regions corresponding to the barrier ribs 212 formed over the rear substrate 211.

FIG. 3 is a perspective view illustrating a structure of discharge cells of a plasma display panel according to an embodiment of the present invention as defined in claim 1.

The discharge cells of the plasma display panel are partitioned by barrier ribs. The barrier ribs each comprises a first barrier rib 212a and a second barrier rib 212b, which intersect with each other and make up one discharge cell. The first barrier ribs 212a partition the discharge cells emitting the same color of light. The second barrier ribs 212b partition the discharge cells emitting different colors of light. In this case, the area of the unit discharge cell and the area of the unit discharge space in the discharge cells may be defined.

The area of the unit discharge space may be defined as the multiplication of a first distance d1 between top portions of the two neighboring first barrier ribs 212a and a second distance d2 between top portions of the two neighboring second barrier ribs 212b. The area of the unit discharge cell may be defined as the multiplication of a third distance d3 between central points of the top portions of the first barrier ribs 212a and a fourth distance d4 between central points of the top portions of the second barrier ribs 212b.

Also, the total area of the discharge cells and the total area of the discharge spaces may be defined in an effective region of the plasma display panel on which an image is displayed.

In other words, the total area of the discharge spaces may be defined by the total addition of the unit discharge spaces, and the total area of the discharge cells may be defined by the total addition of the unit discharge cells.

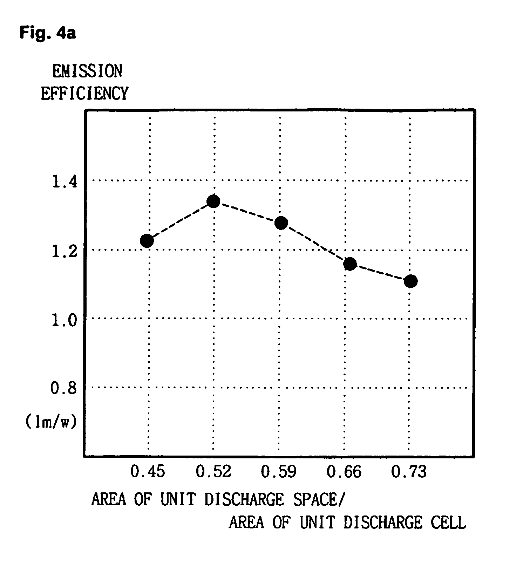

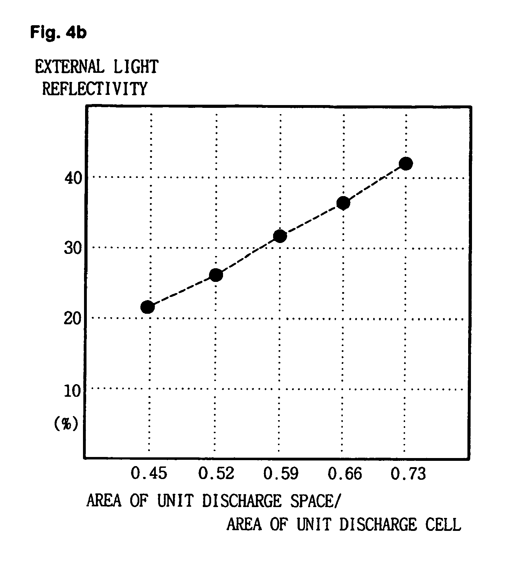

In such plasma display panel with the above described discharge cell structure, emission efficiency based on a ratio between the area of the unit discharge space and the area of the unit discharge cell may be obtained as in the following exemplary experimental examples and FIGS. 4a and 4b.

[Exemplary Experimental Examples]

| | | | | | |
|---|---|---|---|---|---|
| Width of second barrier rib | 60µm | 120µm | 180µm | 240µm | 300µm |
| Width of black layer | 60µm | 120µm | 180µm | 240µm | 300µm |
| Area of unit discharge space | 620×240= 149Kµm² | 560×240= 134Kµm² | 500×240= 120Kµm² | 440×240= 106Kµm² | 380×240= 91Kµm² |
| Area of unit discharge space/Area of unit discharge cell | 149/204= 0.73 | 134/204= 0.66 | 120/204= 0.59 | 106/204= 0.52 | 91/204= 0.45 |
| Emission efficiency (Im/w) | 1.11 | 1.17 | 1.26 | 1.35 | 1.23 |
| External light reflectivity of lasma display panel (%) | 42.8 | 37.4 | 32.3 | 27.1 | 21.9 |

The exemplary experimental examples and FIGS. 4a and 4b show the external light reflectivity of the plasma display panel when the first barrier rib had a fixed width of about 60 m, and the widths of the second barrier rib and the black layer were changed, thereby changing the area of the unit discharge space. In one example the area of the unit discharge cell was about 204K µm² , which is the multiplication value of the third distance d3 and the fourth distance d4. The third distance d3 and the fourth distance d4 were about 680 µm and 300 µm, respectively.

As described above, the emission efficiency and external light reflectivity of the plasma display panel increased when the ratio between the area of the unit discharge space and the area of the unit discharge cell was greater than about 0.45 and less than about 0.59.

The emission efficiency and external light reflectivity of the plasma display panel increased when the ratio of the total area of the unit discharge space to the total area of the unit discharge cell was greater than about 0.45 and less than about 0.59.

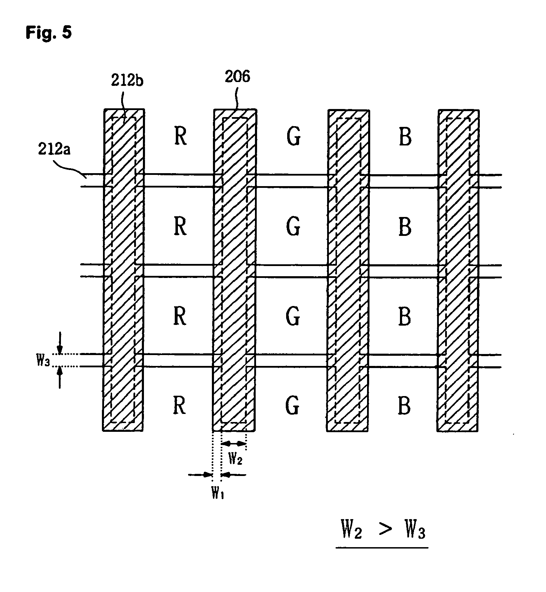

As illustrated in FIG. 5, black layers 206 may be formed substantially in the same regions where second barrier ribs 212b are formed so as to prevent a decrease in brightness. The black layers 206 are positioned to correspond to a portion of the inside of the respective unit discharge cell spaces so as to improve a contrast characteristic by absorbing external light.

In this case, the brightness and contrast characteristics are improved when the width w1 of each of the black layers 206 positioned to correspond to the portion of the inside of the unit discharge space is less than the width w2 of a top portion of the corresponding second barrier rib 212b by about 0.2-fold.

As described above, the discharge cells comprise red discharge cells, green discharge cells and blue discharge cells. Each of the discharge cells comprises red, green and blue discharge spaces defined by the top portions of the barrier ribs. At least one of the red, green and blue discharge spaces may have the width different from the widths of the rest discharge spaces. In detail, the green discharge spaces or the blue discharge spaces may be wider than the red discharge spaces.

The above described discharge space structure may be obtained according to a change in the width of the second barrier ribs partitioning the unit discharge cells, which emit different colors of light. More specifically, the width w2 of the top portion of each of the second barrier rib 212b may vary within in a range of about 3-fold to 5-fold greater than the width w3 of the top portion of each of the first barrier ribs 212a.

The variation in the area of each discharge space contributes to improvement in color temperature of the plasma display panel.

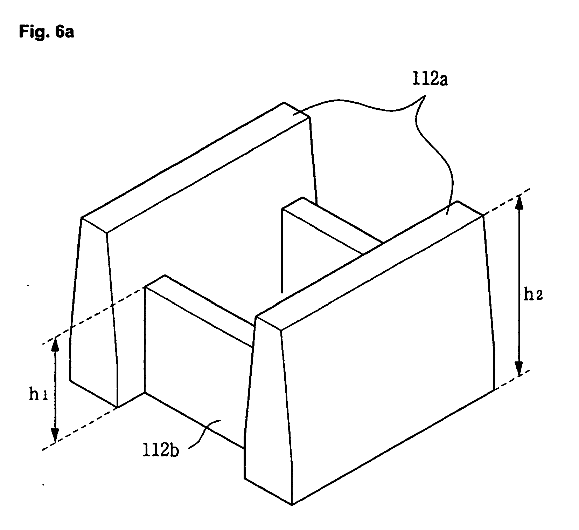

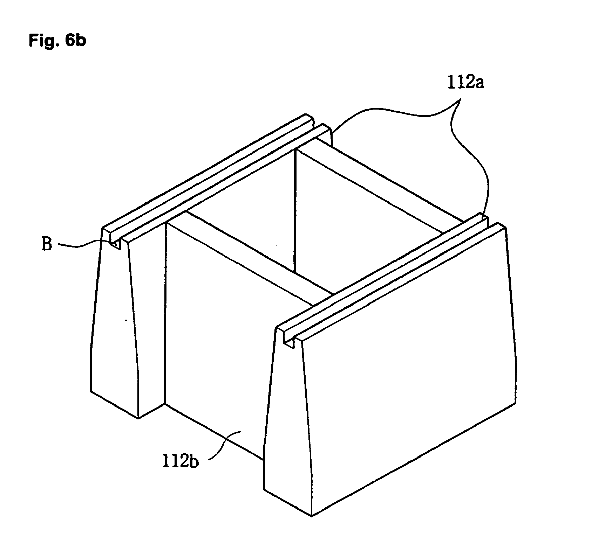

FIGS. 6a and 6b are perspective views illustrating a structure of a barrier rib of a plasma display panel according to an embodiment of the present invention as defined in claim 1.

Referring to FIG. 6a, the barrier rib 112 comprises first barrier ribs 112a and second barrier ribs 112b. The height h2 of the first barrier ribs 112a is different from the height h1 of the second barrier ribs 112b. In this case, the first barrier ribs 112a higher than the second barrier rib 112b partition discharge cells where phosphors with different colors are formed, and the second barrier ribs 112b relatively lower than the first barrier ribs 112a partition discharge cells where phosphors with the same color are formed.

This barrier rib structure improves an exhaust characteristic during the fabrication of plasma display panels, and prevents an event of color mixing in phosphors, which may occur between neighboring discharge cells when coating the phosphors over the discharge cells.

Referring to FIG. 6b, long indentations B are formed lengthwise in top portions of first barrier ribs 112a or second barrier ribs 112b. This barrier rib structure can improve an exhaust characteristic of a plasma display panel.

## Claims

1. A plasma display panel comprising:
a front substrate (201);
a rear substrate (211) formed in parallel to the front substrate;
a first barrier rib (212a; 112a) and a second barrier rib (212b; 112b), the first barrier rib formed between the front substrate and the rear substrate in a first direction, the second barrier rib formed in a second direction intersecting with the first direction;
a black layer (206) formed on the front substrate corresponding to the first barrier rib,
wherein the first barrier rib and the second barrier rib partition a plurality of unit discharge cells such that the first barrier rib partitions unit discharge cells emitting the same color of light, such that the second barrier rib partitions unit discharge cells emitting different colors of light, and such that a unit discharge space inside one of the unit discharge cells is defined, and
the ratio of the area of the unit discharge space to the area of the unit discharge cell ranges from about 0.45 to about 0.59,
wherein the area of the unit discharge space is equal to a first distance (d₁) between top portions of the two neighboring first barrier ribs multiplied by a second distance (d₂) between top portions of the two neighboring second barrier ribs, and
wherein the area of the unit discharge cell is equal to a third distance (d₃) between central points of widths of top portions of the two neighboring first barrier ribs multiplied by a fourth distance (d₄) between central points of widths of top portions of the two neighboring second barrier ribs,
**characterized in that**
the black layer is positioned to correspond to a portion of the inside of the unit discharge space, and
the width (W₁) of the black layer positioned to correspond to the portion of the inside of the unit discharge space is less than the width (W₂) of the top portion of the second barrier rib by 0.2 times.

2. The plasma display panel of claim 1, wherein at least one groove is formed in a top portion of the first barrier rib or the second barrier rib in a direction of length of the first barrier rib or of the second barrier rib.

3. The plasma display panel of claim 1, wherein the black layer is formed to integrate with the bus electrode (202b, 203b) formed over the front substrate.

4. The plasma display panel of claim 1,
wherein the first barrier rib and the second barrier rib define a plurality of the unit discharge cells and a plurality of the unit discharge spaces,
wherein the total area of the discharge spaces is obtained by adding the areas of unit discharge spaces together,
wherein the total area of the discharge cells is obtained by adding the areas of unit discharge cells together, and
wherein the ratio of the total area of the discharge spaces to the total area of the discharge cells ranges from about 0.45 to about 0.59 in an effective region on which an image is displayed.

5. The plasma display panel of claim 4, wherein the discharge spaces comprise a red discharge space (R), a green discharge space (G) and a blue discharge space (B), wherein at least one of the red, green and blue discharge spaces has a width different from a width of the remaining discharge spaces.

6. The plasma display panel of claim 5, wherein at least one of the blue discharge space and the green discharge space has a width greater than that of the red discharge space.

7. The plasma display panel of claim 4, wherein at least one indentation is formed in a top portion of the first barrier rib or the second barrier rib in a direction of length of the first barrier rib or of the second barrier

## Patentansprüche

1. Plasmaanzeigepaneel, umfassend:
ein Frontsubstrat (201);
ein Rücksubstrat (211), das parallel zu dem Frontsubstrat gebildet ist;
eine erste Begrenzungsrippe (212a; 112a) und eine zweite Begrenzungsrippe (212b; 112b), wobei die erste Begrenzungsrippe zwischen dem Frontsubstrat und dem Rücksubstrat in einer ersten Richtung gebildet ist und die zweite Begrenzungsrippe in einer zweiten Richtung gebildet ist, die die erste Richtung schneidet;
eine schwarze Schicht (206), die auf dem Frontsubstrat in Entsprechung mit der ersten Begrenzungsrippe ausgebildet ist,
wobei die erste Begrenzungsrippe und die zweite Begrenzungsrippe eine Vielzahl von Einheitsentladezellen derart abteilt, dass die erste Begrenzungsrippe Einheitsentladezellen abteilt, die die gleiche Lichtfarbe abstrahlen, derart, dass die zweite Begrenzungsrippe Einheitsentladezellen abteilt, die Licht unterschiedlicher Farben abstrahlen, und derart, dass ein Einheitsentladeraum innerhalb einer der Einheitsentladezellen definiert wird, und
das Verhältnis der Fläche des Einheitsentladeraums zu der Fläche der Einheitsentladezelle von etwa 0,45 bis etwa 0,59 reicht,
wobei die Fläche des Einheitsentladeraums gleich einem ersten Abstand (d₁) zwischen Spitzenabschnitten der zwei benachbarten ersten Begrenzungsrippen multipliziert mit einem zweiten Abstand (d₂) zwischen Spitzenabschnitten der zwei benachbarten zweiten Begrenzungsrippen ist, und
wobei die Fläche der Einheitsentladezelle gleich einem dritten Abstand (d₃) zwischen Mittelpunkten der Breiten von Spitzenabschnitten der zwei benachbarten ersten Begrenzungsrippen multipliziert mit einem vierten Abstand (d₄) zwischen Mittelpunkten der Breiten von Spitzenabschnitten der zwei benachbarten zweiten Begrenzungsrippen ist,
**dadurch gekennzeichnet, dass**
die schwarze Schicht positioniert ist, um einem Abschnitt des Inneren der Einheitsentladezelle zu entsprechen, und
die Breite (W₁) der schwarzen Schicht, die positioniert ist, um dem Abschnitt des Inneren der Einheitsentladezelle zu entsprechen, um das 0,2-fache kleiner als die Breite (W₂) des Spitzenabschnitts der zweiten Begrenzungsrippe ist.

2. Plasmaanzeigepaneel gemäß Anspruch 1, wobei zumindest eine Ausnutzung in einem Spitzenabschnitt der ersten Begrenzungsrippe oder der zweiten Begrenzungsrippe in einer Längenrichtung der ersten Begrenzungsrippe oder der zweiten Begrenzungsrippe gebildet ist.

3. Plasmaanzeigepaneel gemäß Anspruch 1, wobei die schwarze Schicht ausgebildet ist, um sich mit der Buselektrode (202b, 203b) zu integrieren, die über dem Frontsubstrat gebildet ist.

4. Plasmaanzeigepaneel gemäß Anspruch 1,
wobei die erste Begrenzungsrippe und die zweite Begrenzungsrippe eine Vielzahl der Einheitsentladezellen und eine Vielzahl der Einheitsentladeräume definieren,
wobei die Gesamtfläche der Entladeräume durch Addieren der Flächen der Einheitsentladeräume erhalten wird,
wobei die Gesamtfläche der Entladezellen durch Addieren der Flächen der Einheitsentladezellen erhalten wird, und
wobei das Verhältnis der Gesamtfläche der Entladeräume zu der Gesamtfläche der Entladezellen von etwa 0,45 bis etwa 0,59 in einem effektiven Bereich reicht, auf dem ein Bild angezeigt wird.

5. Plasmaanzeigepaneel gemäß Anspruch 4, wobei die Entladeräume einen roten Entladeraum (R), einen grünen Entladeraum (G) und einen blauen Entladeraum (B) umfassen, wobei zumindest einer des roten, grünen und blauen Entladeraums eine Breite aufweist, die von einer Breite der verbleibenden Entladeräume verschieden ist.

6. Plasmaanzeigepaneel gemäß Anspruch 5, wobei zumindest einer des blauen Entladeraums und des grünen Entladeraums eine Breite aufweist, die größer als jene des roten Entladeraums ist.

7. Plasmaanzeigepaneel gemäß Anspruch 4, wobei zumindest eine Einbuchtung in einem Spitzenabschnitt der ersten Begrenzungsrippe oder der zweiten Begrenzungsrippe in einer Längenrichtung der ersten Begrenzungsrippe oder der zweiten Begrenzungsrippe gebildet ist.

## Revendications

1. Panneau d'affichage au plasma comprenant :
un substrat avant (201) ;
un substrat arrière (211) formé en parallèle au substrat avant ;
une première nervure barrière (212a ; 112a) et une deuxième nervure barrière (212b ; 112b), la première nervure barrière formée entre le substrat avant et le substrat arrière dans un premier sens, la deuxième nervure barrière formée dans un deuxième sens coupant le premier sens ;
une couche noire (206) formée sur le substrat avant correspondant à la première nervure barrière,
dans lequel la première nervure barrière et la deuxième nervure barrière partagent une pluralité de cellules de décharge unitaires de telle manière que la première nervure barrière partage des cellules de décharge unitaires émettant le même couleur de lumière, de telle manière que la deuxième nervure barrière partage des cellules de décharge unitaires émettant différentes couleurs de lumière, et de telle manière qu'un espace de décharge unitaire à l'intérieur d'une des cellules de décharge unitaires est défini, et
le rapport de la superficie de l'espace de décharge unitaire sur la superficie de la cellule de décharge unitaire est dans la plage d'environ 0,45 à environ 0,59,
dans lequel la superficie de l'espace de décharge unitaire est égale à une première distance (d₁) entre des parties supérieures des deux premières nervures barrière voisines multipliée par une deuxième distance (d₂) entre des parties supérieures des deux deuxièmes nervures barrière voisines, et
dans lequel la superficie de la cellule de décharge unitaire est égale à une troisième distance (d₃) entre des points centraux de largeurs de parties supérieures des deux premières nervures barrière voisines multipliée par une quatrième distance (d₄) entre des points centraux de largeurs de parties supérieures des deux deuxièmes nervures barrière voisines,
**caractérisé en ce que**
la couche noire est positionnée de façon à correspondre à une partie de l'intérieur de l'espace de décharge unitaire, et
la largeur (W₁) de la couche noire positionnée de façon à correspondre à la partie de l'intérieur de l'espace de décharge unitaire est inférieure à la largeur (W₂) de la partie supérieure de la deuxième nervure barrière de 0,2 fois.

2. Panneau d'affichage au plasma selon la revendication 1, dans lequel au moins une rainure est formée dans une partie supérieure de la première nervure barrière ou de la deuxième nervure barrière dans un sens de la longueur de la première nervure barrière ou de la deuxième nervure barrière.

3. Panneau d'affichage au plasma selon la revendication 1, dans lequel la couche noire est formée de façon à s'intégrer avec l'électrode de bus (202b, 203b) formée par-dessus le substrat avant.

4. Panneau d'affichage au plasma selon la revendication 1,
dans lequel la première nervure barrière et la deuxième nervure barrière définissent une pluralité des cellules de décharge unitaires et une pluralité des espaces de décharge unitaires,
dans lequel la superficie totale des espaces de décharge est obtenue en additionnant les superficies des espaces de décharge unitaires ensemble,
dans lequel la superficie totale des cellules de décharge est obtenue en additionnant les superficies des cellules de décharge unitaires ensemble, et
dans lequel le rapport de la superficie totale des espaces de décharge sur la superficie totale des cellules de décharge est dans la plage d'environ 0,45 à environ 0,59 dans une région utile sur laquelle une image est affichée.

5. Panneau d'affichage au plasma selon la revendication 4, dans lequel les espaces de décharge comprennent un espace de décharge rouge (R), un espace de décharge verte (V) et un espace de décharge bleue (B), dans lequel au moins un des espaces de décharge rouge, verte et bleue a une largeur différente d'une largeur des espaces de décharge restants.

6. Panneau d'affichage au plasma selon la revendication 5, dans lequel au moins un de l'espace de décharge bleue et de l'espace de décharge verte a une largeur plus grande que celle de l'espace de décharge rouge.

7. Panneau d'affichage au plasma selon la revendication 4, dans lequel au moins une indentation est formée dans une partie supérieure de la première nervure barrière ou de la deuxième nervure barrière dans un sens de la longueur de la première nervure barrière ou de la deuxième nervure barrière.
